# EUROPÄISCHE PATENTSCHRIFT

(11) **EP 4 434 466 B1**
(45) Veröffentlichungstag und Bekanntmachung des Hinweises auf die Patenterteilung: **30.07.2025**
(21) Anmeldenummer: 23163685.3
(22) Anmeldetag: 23.03.2023
(51) Int. Cl.: A61B 6/03, A61B 6/00

(54) **VERFAHREN ZUM BETRIEB EINER COMPUTERTOMOGRAPHIEEINRICHTUNG UND COMPUTERTOMOGRAPHIEEINRICHTUNG**
METHOD FOR OPERATING A COMPUTER TOMOGRAPHY DEVICE AND COMPUTER TOMOGRAPHY DEVICE
PROCÉDÉ DE FONCTIONNEMENT D'UN DISPOSITIF DE TOMOGRAPHIE ASSISTÉE PAR ORDINATEUR ET DISPOSITIF DE TOMOGRAPHIE ASSISTÉE PAR ORDINATEUR

(43) Veröffentlichungstag der Anmeldung: 25.09.2024
(73) Patentinhaber: Siemens Healthineers AG, 91301 Forchheim (DE)
(72) Erfinder: Kreisler, Björn, 91353 Hausen (DE); Schweikert, Benjamin, 91468 Gutenstetten (DE); Wolz, Florian, 91058 Erlangen (DE)
(74) Vertreter: Siemens Healthineers Patent Attorneys

(56) Entgegenhaltungen:
- EP-A1- 3 760 126
- DE-A1- 19 837 442
- US-A1- 2017 042 494

## Beschreibung

Die Erfindung betrifft ein Verfahren zum Betrieb einer Computertomographieeinrichtung, welche einen rotierbaren Anteil mit wenigstens einem Röntgendetektor und einen gegenüber dem rotierbaren Anteil feststehenden Anteil mit einer Recheneinrichtung, die zur Verarbeitung von mit dem wenigstens einen Röntgendetektor aufgenommenen Detektordaten ausgebildet ist, aufweist, wobei dem wenigstens einen Röntgendetektor eine Aufbereitungseinrichtung zur Ermittlung von Detektordaten, die über einen Kommunikationsweg zu der Recheneinrichtung zu übertragen sind, aus Rohdaten des wenigstens einen Röntgendetektors zugeordnet ist und der Kommunikationsweg eine drahtlose Kommunikationsstrecke mit einer Maximalübertragungsrate aufweist. Daneben betrifft die Erfindung eine Computertomographieeinrichtung.

Computertomographieeinrichtungen sind im Stand der Technik bereits hinreichend bekannt und weisen üblicherweise wenigstens eine um einen Patienten oder sonstiges zu untersuchendes Objekt rotierbare Aufnahmeanordnung mit einem Röntgenstrahler und einem Röntgendetektor auf. Die Aufnahmeanordnung mit dem Röntgenstrahler und dem Röntgendetektor gehört somit zu einem rotierbaren Anteil der Computertomographieeinrichtung, welcher beispielsweise in einer Gantry der Computertomographieeinrichtung, die zum feststehenden Anteil gehört, geführt sein kann. Um Computertomographiebilddatensätze ermitteln zu können, werden mit dem wenigstens einen Röntgendetektor aufgenommene Rohdaten üblicherweise vor Ort mittels einer Aufbereitungseinrichtung zu Detektordaten aufbereitet, die an eine Recheneinrichtung der Computertomographieeinrichtung übertragen werden, die zum feststehenden Anteil gehört und häufig auch als Bildrechner bezeichnet wird. Der Bildrechner nutzt die Detektordaten zur Rekonstruktion eines Computertomographiebilddatensatzes, beispielsweise eines Satzes von Schnittbildern und/oder eines dreidimensionalen Bildvolumens.

Um möglichst weitgehende Bewegungsfreiheit des rotierbaren Anteils sicherzustellen, insbesondere Rotationsläufe über große Winkelintervalle, beispielsweise komplette Umläufe, zu erlauben, wurde vorgeschlagen, auf dem Kommunikationsweg von der Aufbereitungseinrichtung zu der Recheneinrichtung eine drahtlose Kommunikationsstrecke vorzusehen. Technisch bedingt ist die Maximalübertragungsrate der drahtlosen Kommunikationsstrecke allerdings begrenzt, so dass beispielsweise maximal 25 bis 30 Gbit/s übertragen werden können. Aktuelle Röntgendetektoren, insbesondere sogenannte zählende Röntgendetektoren (Counting Detectors), erzeugen intern Rohdaten mit einer deutlich höheren Datenrate, insbesondere dann, wenn mehrere Röntgendetektoren vorgesehen sind. Dies kann beispielsweise dann der Fall sein, wenn die Computertomographieeinrichtung eine Biplan-Computertomographieeinrichtung ist, bei der der rotierbare Anteil zwei in einem Winkel zueinander stehende Aufnahmeanordnungen mit jeweiligen Röntgendetektoren aufweist. Beispielsweise können sich bei zwei zählenden Röntgendetektoren Rohdatenraten von mehreren hundert Gbit/s ergeben.

Bei Computertomographieeinrichtungen beziehungsweise allgemein bei Röntgeneinrichtungen besteht die Anforderung, dass applizierte Röntgenstrahlung in jedem Fall in ein diagnosefähiges Röntgenbild übersetzt werden muss. Das bedeutet, auch bei einem Systemausfall, beispielsweise einem Stromausfall, einem Fehler entlang des Kommunikationswegs und/oder einem sonstigen Abbruch, müssen aufgenommene Detektordaten bereits persistent in der Recheneinrichtung gespeichert sein und eine Rekonstruktion eines Computertomographiedatensatzes muss möglich sein. Das bedeutet, es muss eine Echtzeitübertragung der Detektordaten sichergestellt werden.

Um dies zu ermöglichen, verarbeitet die Aufbereitungseinrichtung bei bekannten Computertomographieeinrichtungen die Rohdaten derart, dass ein Datenstrom aus Detektordaten entsteht, dessen Übertragungsrate (Datenrate) kleiner oder gleich der Maximalübertragungsrate ist. Hierzu ist es beispielsweise bekannt, die räumliche Auflösung seitens der Aufbereitungseinrichtung zu reduzieren, bei zählenden Röntgendetektoren nur einer geringen Anzahl von Energieschwellwerten zugeordnete Detektordaten auszuwählen und zu übertragen, und/oder Kompressionsverfahren einzusetzen. Zum Letzteren gehören beispielsweise hybrides Datencoding und differenzielles statistisches Datencoding. Zur Reduzierung der räumlichen Auflösung ist es beispielsweise bekannt, immer vier Pixel am Röntgendetektor (2x2) zusammenzufassen. Dieser Vorgang kann auch als "Fusing" bezeichnet werden.

Auf diese Weise ist es möglich, die Anforderungen an eine Echtzeitübertragung zu erfüllen und einen Datenstrom von Detektordaten zu erzeugen, dessen Übertragungsrate bei unmittelbarer Übertragung (Echtzeitübertragung) niedriger oder gleich der Maximalübertragungsrate der drahtlosen Kommunikationsstrecke ist. Nach jedem Zeitschritt, in dem der wenigstens eine Röntgendetektor ausgelesen wird, kann gemessene Information in sehr kleinen Paketen gesendet und auf dem Bildrechner, also der Recheneinrichtung, gespeichert werden.

Sollten kurzzeitige Übertragungsfehler auftreten, wurde vorgeschlagen, einen FIFO-Pufferspeicher zu verwenden, um ein erneutes Senden (resend) zu ermöglichen. Hierdurch kann es zwar zu kleineren Verzögerungen kommen, jedoch liegen bei einem Abbruch der Rohdatenerfassung die bislang gemessenen Detektordaten bis auf gegebenenfalls sehr wenige kleine Datenpakete schon auf der Recheneinrichtung vor.

Dies führt bei bekannten Computertomographieeinrichtungen zu starken Einschränkungen, wenn hochgenaue Informationen gefordert sind. So ist es beispielsweise bekannt, dass, um die hohe räumliche Auflösung des wenigstens einen Röntgendetektors auch nutzen zu können, die Zahl übertragener Detektorzeilen im Gegenzug eingeschränkt wird, um die Maximalübertragungsrate einzuhalten. Die Nutzung weiterer Energieschwellwerte des wenigstens einen Röntgendetektors kann beispielsweise dazu führen, dass die räumliche Auflösung deutlicher reduziert werden muss.

DE 198 37 442 A1 betrifft ein CT-Gerät mit einem im Betrieb des CT-Gerätes rotierenden Geräteteil, auf dem außer einem Detektorsystem zur Ermittlung von Messdaten alle Komponenten, die zur Erzeugung von Bilddaten aus den Messdaten erforderlich sind, angeordnet sind. Hieraus resultiert eine verminderte Datenübertragungsrate bei der Datenübertragung von dem rotierenden auf ein stationäres Geräteteil, mit der Folge, dass der im Zusammenhang mit der Datenübertragung zu treibende Aufwand vermindert ist.

US 2017/042494 A1 offenbart eine CT-Einrichtung mit einer Rekonstruktionseinheit, die ein erstes CT-Bild eines Sichtfelds unter Verwendung eines ersten Sinogramms rekonstruiert, und einer Korrektureinheit, die ein zweites Sinogramm durch Vorwärtsprojektion aus den ersten CT-Bild ermittelt sowie ein zweites CT-Bild rekonstruiert, wozu das zweite Sinogramm und ein drittes Sinogramm, das einen Anteil eines Objekts außerhalb des Sichtfelds beschreibt, verwendet werden. Das dritte Sinogramm kann durch Modellierung des fehlenden Anteils bestimmt werden. So wird ein CT-Bild höherer Qualität bereitgestellt.

EP 3 760 126 A1 offenbart Verfahren und Systeme zur Hochqualitäts-CT-Bildgebung. Ein erster Datensatz wird erzeugt, indem mit einem Detektor-Array individueller Detektoren mit einer ersten Zahl von Energie-Bins aufgenommene Photonen für ein virtuelles Detektor-Array durch Aggregation von individuellen Detektoren zu Makro-Detektoren mit einer zweiten Zahl von Energie-Bins zusammengefasst werden. Ein zweiter Datensatz für ein erweitertes Detektor-Array mit den individuellen Detektoren hinsichtlich der zweiten Zahl von Energie-Bins wird durch Up-Sampling des ersten Datensatzes ermittelt. Aus diesem wird ein Bild rekonstruiert. So können hochauflösende Detektor-Arrays minimale Datenmengen aufnehmen und dennoch eine hohe räumliche und spektrale Auflösung erhalten werden.

Der Erfindung liegt daher die Aufgabe zugrunde, bei Computertomographieeinrichtungen auch datenintensivere Bildgebungsvorgänge trotz der Nutzung einer drahtlosen Kommunikationsstrecke und der Anforderung eines auch bei Abbruch beziehungsweise Systemausfall vorliegenden Computertomographiebildes zu erlauben.

Zur Lösung dieser Aufgabe sind erfindungsgemäß ein Verfahren mit den Merkmalen des Anspruchs 1 und eine Computertomographieeinrichtung mit den Merkmalen des Anspruch 14 vorgesehen.

Bei einem Verfahren der eingangs genannten Art ist erfindungsgemäß vorgesehen, dass bei einem Bildgebungsvorgang
- mittels einer Selektionseinheit der Aufbereitungseinrichtung die Detektordaten in einen ersten Datenstrom, dessen erste Detektordaten nach Abschluss der Rohdatenerfassung mit dem wenigstens einen Röntgendetektor zur Rekonstruktion eines auswertbaren Computertomographiebilddatensatzes ausreichend sind, mit einer Datenübertragungsrate, die höchstens der Maximalübertragungsrate entspricht, und einen zweiten Datenstrom, der als zweite Detektordaten die restlichen Detektordaten umfasst, aufgeteilt wird,
- der erste Datenstrom als Echtzeitübertragung unmittelbar über den Kommunikationsweg an die Recheneinrichtung übermittelt wird und
- die zweiten Detektordaten des zweiten Datenstroms in einer Zwischenspeichereinrichtung zwischengespeichert und, nach Abschluss der Rohdatenerfassung mit dem wenigstens einen Röntgendetektor und der Übertragung des ersten Datenstroms, über den Kommunikationsweg an die Recheneinrichtung übertragen werden,
- wobei die Selektionseinheit (24) die Aufteilung gemäß wenigstens einem, von einer Steuereinrichtung (17) der Computertomographieeinrichtung (10) vorgebbaren, Selektionsparameter vornimmt.

Hierbei findet das Speichern der Detektordaten des zweiten Datenstroms im Zwischenspeicher selbstverständlich auch in Echtzeit statt, so dass der in dem wenigstens einen Röntgendetektor entstehende Rohdatenstrom in Echtzeit durch die Aufbereitungseinrichtung verarbeitet und die entstehenden Detektordaten mittels der Selektionseinheit in die Datenströme aufgeteilt werden können. Es entsteht mithin ein kontinuierlicher Echtzeit-Datendurchfluss. Dabei wäre die nötige Datenübertragungsrate zur ungepufferten Übertragung (Echtzeit-Übertragung) des ersten und des zweiten Datenstroms gemeinsam größer als die Maximalübertragungsrate. Insbesondere ist es sogar denkbar, dass die Datenübertragungsrate für eine ungepufferte Übertragung (Echtzeit-Übertragung) des zweiten Datenstroms alleine größer als die Maximalübertragungsrate ist.

Allgemein gesprochen kann die Aufbereitungseinrichtung insbesondere auch zur Kompression von Detektordaten mittels einer Kompressionsmethode ausgebildet sein, wie dies im Stand der Technik grundsätzlich bekannt ist. Die Aufbereitungseinrichtung bereitet die Detektordaten aber auch anderweitig auf die Übertragung vor, insbesondere durch Auswahl und/oder Formatierung und/oder sonstige Vorverarbeitung. Die Aufbereitungseinrichtung kann ferner zur Anpassung einer räumlichen und/oder zeitlichen Auflösung von Detektordaten ausgebildet sein und/oder zur Ermittlung einer Frequenzdarstellung der Detektordaten, worauf im Folgenden noch genauer eingegangen werden wird.

Der Erfindung liegt der Gedanke zugrunde, die Detektordaten derart vorzusortieren, dass für die Ermittlung eines Computertomographiebilddatensatzes ausreichende, wichtige erste Detektordaten zunächst unmittelbar ungepuffert an die Recheneinrichtung, also den Bildrechner, übertragen werden und dort gespeichert werden, während zweiten Detektordaten eine geringere Wichtigkeit hinsichtlich der Anforderung des Vorliegens von hinreichenden Detektordaten zur Rekonstruktion eines Computertomographiedatensatzes zugeordnet wird und diese zunächst zwischengespeichert werden, um im Nachlauf übertragen zu werden. Das bedeutet, die Übertragung der ersten Detektordaten im ersten Datenstrom und die Übertragung der zweiten Detektordaten wird nacheinander durchgeführt. Auf diese Weise kann sichergestellt werden, dass zu jedem Zeitpunkt eine vollständige Messung erfolgt und auch zur Ermittlung eines Computertomographiedatensatzes ausreichende Anteile sofort an die Recheneinrichtung gesendet und dort gespeichert werden. Dass die ersten Detektordaten nach Abschluss der Rohdatenerfassung mit dem wenigstens einen Röntgendetektor zur Rekonstruktion eines auswertbaren Computertomographiebilddatensatzes ausreichend sind, kann so zu verstehen sein, dass sie ein vorgegebenes Minimaldatenkriterium erfüllen. Weitere Information wird erst nach dem Ende der Rohdatenerfassung (und der Übertragung der ersten Detektordaten) an die Recheneinrichtung gesendet. Dieser Nachlauf erhöht die Datenmenge und damit auch den Informationsinhalt der verfügbaren Detektordaten. Durch die vorgenommene Selektion und die zeitliche Verzögerung können somit mehr Detektordaten übertragen werden, wobei trotzdem direkt zum Ende der Rohdatenerfassung (Scan-Ende) ein vollständiger Computertomographiebilddatensatz für die Diagnose verfügbar ist. Weitere Information, wie beispielsweise höhere räumliche Auflösung oder weitere spektrale Information, ist verzögert verfügbar und kann entsprechend in der Folge genutzt werden.

Um diese Vorsortierung der Detektordaten durchzuführen, wird vorgeschlagen, direkt in der Aufbereitungseinrichtung anders zu agieren, so dass eine Selektionseinheit vorgeschlagen wird, um zwei unterschiedliche Datenströme zu erzeugen. Der erste Datenstrom ist für den direkten Transfer an den Bildrechner, also die Recheneinrichtung, vorgesehen, während der zweite Datenstrom, beispielsweise mittels einer entsprechenden Zwischenspeichereinheit, zur Zwischenspeicherung in einer Zwischenspeichereinrichtung weitergeleitet wird, um verzögert nach Ende der Rohdatenerfassung transferiert zu werden. Diese beiden Datenströme werden dabei, wie bereits dargelegt, sozusagen synchron befüllt.

Während bisher die Datenrate durch die Maximalübertragungsrate für den unmittelbaren Transfer begrenzt war und höhere Datenraten konsequent verhindert wurden, können durch die hier vorgestellte Lösung Datenraten beziehungsweise Datenmengen ermöglicht werden, die mit aktueller Datenübertragungstechnik noch nicht erreicht werden konnten. Hierfür muss lediglich eine geeignet große Zwischenspeichereinrichtung im rotierbaren Anteil, gegebenenfalls als Teil der Aufbereitungseinrichtung, vorgesehen werden.

Dabei sei an dieser Stelle noch allgemein angemerkt, dass der Röntgendetektor insbesondere ein sogenannter zählender Röntgendetektor sein kann, mithin ein zum Zählen einzelner Photonen ausgebildeter Röntgendetektor. Ein zählender Röntgendetektor kann insbesondere dazu ausgebildet sein, nicht nur einzelne Quanten zu detektieren, sondern zusätzlich auch die Energie des Photons zu quantifizieren, so dass eine spektrale Bildgebung, also eine Bildgebung mit Energieauflösung, ermöglicht wird. Hierzu ist es beispielsweise bekannt, verschiedene Energieschwellwerte im Röntgendetektor zu verwenden. Allgemein gesprochen kann es sich bei der Computertomographieeinrichtung um eine Biplan-Computertomographieeinrichtung handeln, bei der der rotierende Anteil mithin zwei Aufnahmeanordnungen mit jeweils einem Röntgenstrahler und einem Röntgendetektor aufweist. Jene können beispielsweise um 90° gegeneinander verdreht angeordnet sein. In diesem Fall liegen also zwei Röntgendetektoren vor, deren Rohdaten in einer gemeinsamen Aufbereitungseinrichtung oder auch getrennten Aufbereitungseinrichtungen für den Transfer zur Recheneinrichtung aufbereitet werden können.

Hierbei sind im Rahmen der vorliegenden Erfindung, allgemein gesprochen, verschiedene Möglichkeiten denkbar, wie die Selektion von ersten Detektordaten für den ersten Datenstrom konkret vorgenommen werden kann, beispielsweise auch im Hinblick auf eine zuvor vorgenommene Aufbereitung der Aufbereitungseinrichtung. Insbesondere kann im Vergleich zu den gesamten Detektordaten eine räumliche, eine zeitliche und/oder eine spektrale Reduzierung erfolgen. Diese Optionen können selbstverständlich auch miteinander kombiniert eingesetzt werden. Insbesondere bei einer Kombination von solchen Reduzierungstechniken ist es mit besonderem Vorteil möglich, dass die Aufteilung zur möglichst weitgehenden Nutzung der Maximalübertragungsrate vorgenommen wird. Das bedeutet, es kann zweckmäßig sein, bereits bei der Übertragung des ersten Datenstroms die Maximalübertragungsrate so weit wie möglich auszunutzen, um den Nachlauf entsprechend minimieren zu können. Auch in denkbaren Fällen, in denen erste Detektordaten gezielt für den ersten Datenstrom geschaffen und bereitgestellt werden, beispielsweise durch eine Reduzierung der räumlichen Auflösung im Vergleich zu in den zweiten Detektordaten noch enthaltenen entsprechenden Informationen, kann dies zweckmäßig sein. Denn dann wird ein möglichst hochqualitativer Computertomographiebilddatensatz auch dann bereitgestellt, wenn die zweiten Detektordaten nicht mehr übertragen werden.

Konkret kann beispielsweise vorgesehen sein, dass der erste Datenstrom wenigstens teilweise in der räumlichen Auflösung und/oder der räumlichen Abdeckung gegenüber den gesamten Detektordaten, insbesondere auch den zweiten Detektordaten alleine, reduzierte erste Detektordaten umfasst. In Ausführungsbeispielen, die eine solche räumliche Sortierung nutzen, werden mithin Detektordaten (zunächst) weggelassen und/oder Detektordaten miteinander verrechnet, um die räumliche Auflösung zu reduzieren. Im zweitgenannten Fall kann auch vorgesehen sein, dass die Reduzierung der räumlichen Auflösung gezielt zur Schaffung der ersten Detektordaten für den ersten Datenstrom genutzt wird, das bedeutet, es werden zunächst räumlich niedriger aufgelöste Detektordaten gesendet. Aus diesen lässt sich bereits ein diagnostisch verwertbarer Computertomographiebilddatensatz ermitteln. In einem zweiten Schritt können danach räumlich höher aufgelöste zweite Detektordaten nachgeliefert werden. Eine Reduzierung der räumlichen Auflösung kann beispielsweise durch das Zusammenfassen von Detektorwerten einer Gruppe benachbarter Pixel erfolgen, beispielsweise von 2x2 oder 3x3 Pixeln in ein neues Pixel. Entsprechend resultiert eine Reduzierung der Datenmenge um den Faktor vier beziehungsweise den Faktor neun. Beispielsweise sind bereits zählende Röntgendetektoren bekannt geworden, deren Pixelgröße im Bereich von 300 µm liegt, so dass eine Zusammenfassung von neun Pixeln noch immer unterhalb von 1 mm Pixelgröße liegen kann.

Eine weitere Möglichkeit zur Reduzierung der Datenmenge in räumlicher Hinsicht ist das Weglassen bestimmter Daten, beispielsweise einer bestimmten Anzahl von Detektorzeilen und/oder Detektorspalten, deren Detektordaten dann als zweite Detektordaten nachgeliefert werden. Beispielsweise wurde im Stand der Technik für manche Anwendungsfälle bereits vorgeschlagen, für jeden Zeitschritt nur eine bestimmte Anzahl von Detektorzeilen zu übertragen, um die Maximalübertragungsrate nicht zu überschreiten. Der Rest der Daten musste verworfen werden. Im Rahmen der vorliegenden Erfindung können diese restlichen Detektordaten in den zweiten Datenstrom einsortiert werden und zwischengespeichert werden, so dass sie nachgeliefert werden können.

In diesem Zusammenhang sind auch Ausführungsbeispiele denkbar, in denen regelmäßige Auslassungen bezüglich Detektorzeilen und/oder Detektorspalten vorgenommen werden, beispielsweise nur jede n-te Zeile und/oder jede n-te Spalte übertragen wird. Dies eröffnet seitens der Recheneinrichtung, worauf im Folgenden noch näher eingegangen werden wird, die Option, fehlende, also erst zu einem späteren Zeitpunkt übertragene oder verloren gegangene zweite Detektordaten durch Interpolation und/oder Extrapolation abzuschätzen und den Detektordatensatz zumindest abgeschätzt wieder zu vervollständigen.

Dabei muss nicht für jeden Zeitschritt, in dem der wenigstens eine Röntgendetektor ausgelesen wird und neue Rohdaten entstehen und/oder in dem neue Detektordaten aufbereitet werden, dieselbe räumliche Auslassung erfolgen. So kann eine konkrete Ausgestaltung der vorliegenden Erfindung beispielsweise vorsehen, dass eine vorgegebene räumliche Gesamtabdeckung der gesamten Detektordaten in wenigstens zwei Untergruppen zerlegt wird, wobei die Rohdatenerfassung in Zeitschritten erfolgt und in aufeinanderfolgenden Zeitschritten jeweils nur die Detektordaten einer der Untergruppen gemäß einer vorgegebenen Reihenfolge der Untergruppe in den ersten Datenstrom selektiert werden. Beispielsweise können alternierend in Zeitschritten Detektorzeilen mit geraden Zeilennummern und Detektorzeilen mit ungeraden Zeilennummern übertragen werden; denkbar ist auch eine Abwechslung zwischen oberer und unterer Hälfte beziehungsweise linker und rechter Hälfte. Auch weitergehende Unterteilungen in Untergruppen sind selbstverständlich möglich. Ein Beispiel für eine derartige Abtastung ist eine Art "even odd-reading Struktur", bei der in jedem n-ten Zeitschritt, also Reading, eine andere räumliche Struktur übertragen wird und dann im Nachlauf die jeweils ausgelassenen Detektordaten übertragen werden, um die volle Information zu erhalten. Dies kann vorzunehmende Interpolationen verbessern.

Auch eine zeitliche Sortierung ist im Rahmen der vorliegenden Erfindung grundsätzlich denkbar. Hierbei kann mithin vorgesehen sein, dass die zeitliche Auflösung der ersten Detektordaten geringer als die der gesamten Detektordaten ist. Beispielsweise kann auch hier vorgesehen sein, dass die Rohdatenerfassung in Zeitschritten erfolgt, wobei der erste Datenstrom nur Detektordaten jedes n-ten Zeitschritts umfasst und die restlichen Detektordaten dem zweiten Datenstrom zugeordnet werden. In diesem Fall wird sofort nur jedes n-te Reading übertragen und im Nachlauf alle vorerst weggelassenen Detektordaten, so dass die volle zeitliche Auflösung erreicht wird. Dabei sieht eine Ausführungsform vor, dass bei der Übertragung der Detektordaten jedes n-ten Zeitschritts im ersten Datenstrom eine Summe und/oder ein Mittelwert über mehrere, hier n, Zeitschritte ermittelt und übertragen wird.

Ferner ist im Rahmen der vorliegenden Erfindung auch eine frequenzbasierte Sortierung denkbar, um räumliche und/oder zeitliche Variationen zu beachten. Beispielsweise kann konkret vorgesehen sein, dass die Aufbereitungseinrichtung eine Transformationseinheit zur Ermittlung einer Raumfrequenzdarstellung der Detektordaten aufweist, wobei nur Detektordaten mit Raumfrequenzen, die ein Raumfrequenzkriterium erfüllen, in den ersten Datenstrom einsortiert werden. Damit wird vorgeschlagen, beispielsweise Methoden, wie sie auch beim Bildaufbau im Streamen von Videos verwendet werden, auf den Einsatz an einer Computertomographieeinrichtung zu übertragen, wobei zunächst nur bestimmte Raumfrequenzen übertragen werden und die fehlende Information erst nachgelagert übertragen wird. Mithin kann die Selektion beispielsweise basierend auf den vorkommenden Raumfrequenzen (Wavelets beziehungsweise auch Ortsfrequenzen) durchgeführt werden. Beispielsweise ist es denkbar, durch das Raumfrequenzkriterium zu überprüfen, ob die Raumfrequenz unterhalb einer Raumfrequenzschwelle liegt. In diesem Fall werden zunächst nur die niedrigen Raumfrequenzen übertragen, so dass zunächst ein gröberes Bild entsteht, ehe mit dem Nachlauf der zweiten Detektordaten eine Verfeinerung stattfindet. Jedoch sind auch andere Selektionsmöglichkeiten denkbar, die durch das Raumfrequenzkriterium abgebildet werden, so dass beispielsweise aufeinanderfolgende Raumfrequenzintervalle abwechselnd den ersten Detektordaten und den zweiten Detektordaten zugeordnet werden können.

Falls Detektordaten räumlich und/oder zeitlich und/oder frequenzbasiert für den ersten Datenstrom ausgelassen werden, kann, wie bereits angedeutet, vorgesehen sein, dass bei Rekonstruktion eines Computertomographiebilddatensatzes aus den Detektordaten des ersten Datenstroms alleine wenigstens ein Teil der ausgelassenen Detektordaten durch Interpolation und/oder Extrapolation rekonstruiert wird. Dies ist insbesondere auch im Hinblick auf eine Zerlegung hinsichtlich der Raumfrequenzen zweckmäßig, da dann auf Empfängerseite, also seitens der Recheneinrichtung, durch Interpolation sofort ein nutzbarer, ergänzter Detektordatensatz entsteht, in dem abgeschätzte Detektordaten aufgrund nachlaufender Datenteile, also zweiter Detektordaten, ersetzt werden können, so dass mit der Zeit ein vollständiger Detektordatensatz erhalten wird. Die Interpolation kann beispielsweise durch lineare Regression erfolgen, wobei auch komplexere Regressionsmethoden, beispielsweise nichtlineare Regression, eingesetzt werden können. Schließlich ist es auch denkbar, auf künstliche Intelligenz basierende Methoden, beispielsweise "Super-Resolution", einzusetzen, beziehungsweise allgemein gesagt Methoden, die auch beim dynamischen Datenaufbau beim Zoomen auf Landkarten im Internet und/oder bei variablen Datenraten beim Streamen von Videos eingesetzt werden.

Dabei sei an dieser Stelle noch allgemein angemerkt, dass es zweckmäßig sein kann, aus den ersten Detektordaten, gegebenenfalls bereits online während des Erhalts, einen vorläufigen Computertomographiebilddatensatz zu rekonstruieren und diesen anzuzeigen. Auf diese Weise wird auch einem Benutzer vermittelt, dass selbst bei einem Abbruch beziehungsweise Systemausfall bereits für ihn nützliche Daten vorliegen, deren Verbesserung beziehungsweise Erweiterung er mit dem Eintreffen der zweiten Detektordaten dann auch entsprechend visuell nachverfolgen kann.

In einer weiteren Möglichkeit zur Selektierung von Detektordaten kann vorgesehen sein, dass die Computertomographieeinrichtung zur spektralen Bildgebung ausgebildet ist und die Detektordaten unterschiedlichen Energieparameterwerten zugeordnete Teildaten umfassen, wobei der erste Datenstrom nur für einen Teil der mehreren Energieparameterwerte Teildaten enthält. Beispielsweise kann, wie bereits dargelegt wurde, der wenigstens eine Röntgendetektor ein zählender Röntgendetektor sein und die Detektordaten für mehrere Energieschwellwerte als Energieparameterwerte erfassen, wobei der erste Datenstrom als erste Detektordaten nur Teildaten wenigstens eines, insbesondere des niedrigsten, der Energieschwellwerte umfasst. In diesem Fall ist also eine spektrale Sortierung gegeben, wobei bei der Sofort-Übertragung des ersten Datenstroms andere Teildaten übertragen werden als im Nachlauf. Auf diese Weise kann die Energieauflösungsfähigkeit des wenigstens einen Röntgendetektors mittels der nachlaufenden Information, also den zweiten Detektordaten, weitgehender ausgenutzt werden.

Dabei sind selbstverständlich auch Kombinationen dieser Selektionstechniken denkbar. So ist es beispielsweise möglich, dass mittels der Aufbereitungseinrichtung für die Teildaten des niedrigsten Energieschwellwerts die räumliche Auflösung reduziert wird, wobei die entstehenden Detektordaten dann als erste Detektordaten in den ersten Datenstrom einsortiert werden. Die höher aufgelösten Detektordaten des niedrigsten Energieschwellwerts sowie die (gegebenenfalls dennoch in ihrer räumlichen Auflösung reduzierten) Detektordaten weiterer Energieschwellwerte werden als zweite Detektordaten zunächst zwischengespeichert und im Nachlauf übertragen.

Mit besonderem Vorteil ist die Selektion hierbei parametrierbar. Die Erfindung sieht vor, dass die Selektionseinheit die Aufteilung gemäß wenigstens einem, von einer Steuereinrichtung der Computertomographieeinrichtung vorgebbaren Selektionsparameter vornimmt. Beispielsweise können unterschiedlichen Aufnahmeprotokollen unterschiedliche Selektionsparameter zugeordnet werden, die für dieses Aufnahmeprotokoll zum einen im ersten Datenstrom die Maximalübertragungsrate möglichst weitgehend nutzen und zum anderen die für die spezielle Bildgebungsaufgabe gewünschten Grobinformationen mit dem ersten Datenstrom sowie weitere gewünschte Informationen als die zweiten Detektordaten im Nachlauf bereitstellen. Diesbezüglich können in vielen Fällen den Aufnahmeprotokollen im Übrigen auch bereits Aufbereitungsparameter für die Aufbereitungseinrichtung im Allgemeinen zugeordnet sein, die die Ermittlung der Detektordaten und somit die Art der zu ermittelnden Detektordaten festlegen.

Konkret kann dabei vorgesehen sein, dass der wenigstens eine Selektionsparameter gemeinsam mit wenigstens einem die Aufbereitung parametrierenden Aufbereitungsparameter von der Steuereinrichtung an die Aufbereitungseinrichtung übermittelt wird. Dabei kann wenigstens einer des wenigstens einen Aufbereitungsparameters in unmittelbarem Zusammenhang mit der Selektion stehen. Beispielsweise kann zur möglichst weitgehenden Nutzung der Maximalübertragungsrate wenigstens einer des wenigstens einen Aufbereitungsparameters so gewählt werden, dass Detektordaten für den ersten Datenstrom in einer geeigneten Menge pro Zeiteinheit entstehen, beispielsweise durch Ändern der räumlichen Auflösung und/oder Anpassen einer Kompression. Mit anderen Worten sind die gewünschten Informationen für jedes Aufnahmeprotokoll ja bereits im Voraus bekannt und können daher zur entsprechenden Einstellung der Aufbereitungseinrichtung genutzt werden, beispielsweise beim sogenannten "Scanload", in dem auch die Komponenten des rotierenden Anteils der Computertomographieeinrichtung von der Steuereinrichtung entsprechend durch Parametrierung vorbereitet werden.

Insbesondere bedeutet dies, dass jedem auswählbaren, Erfassungsparameter für die Rohdatenerfassung und/oder Aufbereitungsparameter für die Aufbereitung vorgebenden Aufnahmeprotokoll der wenigstens eine entsprechende Selektionsparameter zugeordnet ist und vor Start der Rohdatenerfassung an die Aufbereitungseinrichtung übermittelt und dort eingestellt wird. Beispielsweise kann der wenigstens eine Selektionsparameter gemeinsam mit dem wenigstens einen Erfassungsparameter und/oder dem wenigstens einen Aufbereitungsparameter in einer Look-up-Tabelle der Steuereinrichtung gespeichert sein.

Besonders zweckmäßig ist es, wenn der wenigstens eine Selektionsparameter und/oder eine die Aufteilung in die Datenströme beschreibende Selektionsinformation auch an die Recheneinrichtung übermittelt wird, wo in einem Vorbereitungsschritt die empfangenen Detektordaten des ersten Datenstroms und des zweiten Datenstroms zu einem für nachfolgende Datenverarbeitungsprozesse der Recheneinrichtung als Eingangsdaten geeigneten Detektordatensatz kombiniert werden. Auf diese Weise kann die Recheneinrichtung, also der Bildrechner, informiert werden, auf welche Weise die Detektordaten in den ersten und den zweiten Datenstrom aufgeteilt werden und wie sie ihn erreichen, so dass für nachgelagerte Rekonstruktionsschritte zur Ermittlung des endgültigen Computertomographiebilddatensatzes ein dedizierter Vorbereitungsschritt vorgesehen werden kann, der einen Gesamt-Detektordatensatz in einer durch die nachgelagerten Rekonstruktionsschritte erwarteten Sortierung erzeugt. Auf diese Weise können Computertomographiebilddatensätze auf dieselbe Art und Weise ermittelt werden, unabhängig davon, wie die Selektion und Übertragung abgelaufen sind. Mit anderen Worten benötigen folgende Rechenschritte keine Kenntnis mehr von der "zerwürfelten" Datenübertragung der Detektordaten.

Zweckmäßigerweise kann für Übertragungen über den Kommunikationsweg ein Pufferspeicher verwendet werden, der ein erneutes Senden eines Datenpakets der ersten und/oder zweiten Detektordaten bei einer fehlerhaften Übertragung erlaubt. Das Vorsehen derartiger Pufferspeicher, die ein erneutes Senden (resend) erlauben, ist im Stand der Technik bereits grundsätzlich bekannt. Wie eingangs erläutert, wird der erste Datenstrom in Echtzeit, also sofort, wenn in jedem Zeitschritt neue erste Detektordaten vorliegen, versendet. Dabei handelt es sich üblicherweise um nicht allzu große Datenpakete. Das bedeutet aber auch, dass dann, wenn einmal ein Übertragungsfehler auftritt, es nur zu einem kurzzeitigen zeitlichen Versatz kommt.

In zweckmäßiger Weiterbildung kann der Pufferspeicher als ein hierfür reservierter Teil der Zwischenspeichereinrichtung realisiert werden. Auf diese Weise muss nur eine einzige Speichereinrichtung bereitgestellt werden, die den Speicherplatz als Zwischenspeicher für die zweiten Detektordaten sowie den Pufferspeicher für Datenübertragungen über den Kommunikationsweg bereitstellt. Die Zwischenspeichereinrichtung umfasst also einen kleineren Anteil des dortigen Speicherplatzes, der als echter Puffer für erneutes Senden bei der direkten Übertragung dient, und einen größeren Anteil an Speicherplatz, in dem die zweiten Detektordaten abgelegt und nach dem Ende der Rohdatenerfassung übertragen werden.

In einer Ausgestaltung kann während der Übertragung der Detektordaten des zweiten Datenstroms bereits eine den nächsten Bildgebungsvorgang vorbereitende Information an den rotierenden Anteil übermittelt werden. Hiermit kann zweckmäßigerweise zeiteffizient gearbeitet werden und direkt nach dem Abschluss der Übertragung der zweiten Detektordaten an die Recheneinrichtung mit dem nächsten Bildgebungsvorgang begonnen werden. Eine derartige Handhabung ist immer dann zweckmäßig, wenn eine Datenübertragung über die drahtlose Datenübertragungsstrecke in beide Richtungen parallel erfolgen kann, somit kein Anteil der Maximalübertragungsrate für diesen Vorbereitungsvorgang benötigt wird, oder ein Anteil der Maximalübertragungsrate "frei bleibt".

In vorteilhaften Ausführungsbeispielen kann vorgesehen sein, dass bei einer aufgrund eines Fehlers, insbesondere eines Systemausfalls, nur teilweisen Übertragung der zweiten Detektordaten, die eine Zusatzinformation gegenüber den ersten Detektordaten umfassen, die Recheneinrichtung zur Hinzufügung der Zusatzinformation in dem Computertomographiebilddatensatz wenigstens hinsichtlich des übertragenen Teils ausgebildet ist. Das bedeutet, die Handhabung der Detektordaten auf der Recheneinrichtung, insbesondere auch bezüglich des bereits diskutierten Vorbereitungsschritts, ist derart gewählt, dass auch bei teilweiser Übertragung der zweiten Detektordaten der übertragene Teil noch sinnvoll genutzt werden kann. Beispielsweise kann dann zumindest ein Teil des Computertomographiebilddatensatzes in höherer räumlicher Auflösung ermittelt und dargestellt werden und/oder zusätzliche Spektralinformation kann zumindest in einem Teil des Computertomographiebilddatensatzes angezeigt werden. Entsprechende Techniken zur Detektordatenverarbeitung sind im Stand der Technik grundsätzlich bereits bekannt.

Neben dem Verfahren betrifft die Erfindung auch eine Computertomographieeinrichtung, welche einen rotierbaren Anteil mit wenigstens einem Röntgendetektor und einen gegenüber dem rotierbaren Anteil feststehenden Anteil mit einer Recheneinrichtung, die zur Verarbeitung von mit dem wenigstens einen Röntgendetektor aufgenommenen Detektordaten ausgebildet ist, und eine Steuereinrichtung aufweist, wobei dem wenigstens einen Röntgendetektor eine Aufbereitungseinrichtung zur Ermittlung von über einen Kommunikationsweg zu der Recheneinrichtung zu übertragenden Detektordaten aus Rohdaten des wenigstens einen Röntgendetektors zugeordnet ist und der Kommunikationsweg eine drahtlose Kommunikationsstrecke mit einer Maximalübertragungsrate aufweist. Erfindungsgemäß weist die Aufbereitungseinrichtung auf:
- eine Selektionseinheit zur Aufteilung der Detektordaten eines Bildgebungsvorgangs in einen ersten Datenstrom, dessen erste Detektordaten nach Abschluss der Rohdatenerfassung mit dem wenigstens einen Röntgendetektor zur Rekonstruktion eines auswertbaren Computertomographiebilddatensatzes ausreichend sind, mit einer Datenübertragungsrate, die höchstens der Maximalübertragungsrate entspricht, und einen zweiten Datenstrom, der als zweite Detektordaten die restlichen Detektordaten umfasst, gemäß wenigstens einem, von der Steuereinrichtung vorgebbaren, Selektionsparameter,
- eine Zwischenspeichereinheit zum Zwischenspeichern der zweiten Detektordaten des zweiten Datenstroms in einer Zwischenspeichereinrichtung, und
- eine Übertragungseinheit zur unmittelbaren Echtzeitübertragung des ersten Datenstroms über den Kommunikationsweg an die Recheneinrichtung und, nach Abschluss der Rohdatenerfassung des Bildgebungsvorgangs und der Übertragung der ersten Detektordaten, zur Übertragung der zweiten Detektordaten über den Kommunikationsweg an die Recheneinrichtung.

Sämtliche Ausführungen zum erfindungsgemäßen Verfahren lassen sich analog auf die erfindungsgemäße Computertomographieeinrichtung übertragen, so dass auch mit dieser die bereits genannten Vorteile erhalten werden können. Die Aufbereitungseinrichtung kann hierbei auch noch weitere Funktionseinheiten, insbesondere Aufbereitungseinheiten zur Ermittlung von Detektordaten aus den Rohdaten des wenigstens einen Röntgendetektors, aufweisen. Die Zwischenspeichereinrichtung kann als Teil der Aufbereitungseinrichtung, insbesondere in einem gemeinsamen Gehäuse mit der Aufbereitungseinrichtung, vorgesehen sein. Bei dem wenigstens einen Röntgendetektor kann es sich, wie dargelegt, bevorzugt um einen zählenden Röntgendetektor handeln und/oder die Computertomographieeinrichtung kann als eine Biplan-Computertomographieeinrichtung realisiert sein.

Weitere Vorteile und Einzelheiten der vorliegenden Erfindung ergeben sich aus den im Folgenden beschriebenen Ausführungsbeispielen sowie anhand der Zeichnung. Dabei zeigen:
- Fig. 1: einen Ablaufplan eines Ausführungsbeispiels des erfindungsgemäßen Verfahrens,
- Fig. 2: eine Skizze zum Datenfluss gemäß dem Verfahren der Fig. 1,
- Fig. 3: eine Prinzipskizze einer erfindungsgemäßen Röntgeneinrichtung, und
- Fig. 4: den funktionalen Aufbau der Computertomographieeinrichtung und ihrer Komponenten.

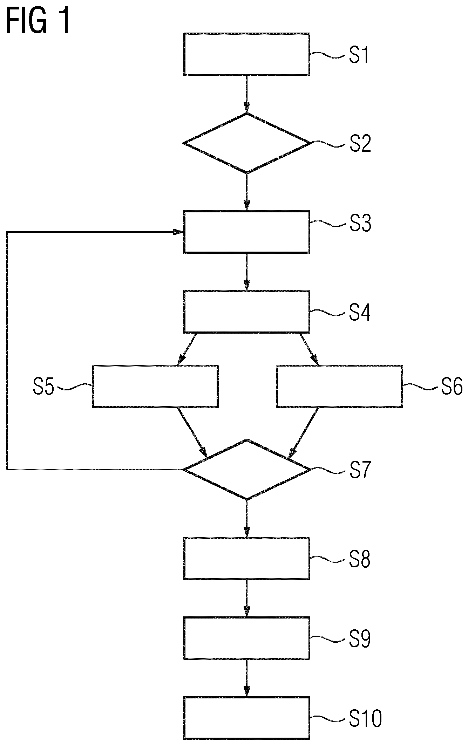

Fig. 1 zeigt einen allgemeinen Ablaufplan eines erfindungsgemäßen Verfahrens zum Betrieb einer Computertomographieeinrichtung bei der Durchführung eines Bildgebungsvorgangs an einem Objekt, hier einem Patienten. Der Patient wird dabei in einer Gantry der Computertomographieeinrichtung derart platziert, dass wenigstens eine Aufnahmeanordnung der Computertomographieeinrichtung, umfassend einen Röntgenstrahler und einen Röntgendetektor, um den Patienten, genauer einen Aufnahmebereich des Patienten, rotiert werden kann. Die Aufnahmeanordnung gehört mithin zu einem rotierbaren Anteil der Computertomographieeinrichtung, der gegen einen feststehenden Anteil, beispielsweise die Gantry, rotiert werden kann. Feststehend sind auch eine als Bildrechner dienende Recheneinrichtung der Computertomographieeinrichtung sowie eine den Betrieb der Computertomographieeinrichtung steuernde Steuereinrichtung, wobei die Recheneinrichtung einen Teil der Steuereinrichtung bilden kann. Um Informationen zur Ansteuerung an die Komponenten des rotierbaren Anteils der Computertomographieeinrichtung übermitteln zu können sowie mit dem wenigstens einen Röntgendetektor erfasste und durch eine Aufbereitungseinrichtung, die auch Teil des rotierbaren Anteils bildet, bereitgestellte Detektordaten an die Recheneinrichtung zu übertragen, ist ein Kommunikationsweg vorgesehen, der vorliegend zur Herstellung idealer rotatorischer Beweglichkeit eine drahtlose Kommunikationsstrecke beinhaltet. Die drahtlose Kommunikationsstrecke weist eine Maximalübertragungsrate auf, das bedeutet, ein Datenstrom mit einer Datenübertragungsrate, die größer als die Maximalübertragungsrate ist, kann nicht in Echtzeit ungepuffert über die drahtlose Kommunikationsstrecke und somit den Kommunikationsweg übertragen werden.

Nichtsdestotrotz ist es bei Computertomographieeinrichtungen eine Anforderung, dass auch bei einem Systemausfall beziehungsweise sonstigem Messabbruch bereits erfasste Detektordaten persistent gespeichert sind und genutzt werden können, um ein diagnostizierbares Computertomographiebild zu rekonstruieren. Das im Folgenden beschriebene Verfahren stellt eine Möglichkeit bereit, diese Anforderung zu erfüllen und gleichzeitig dennoch zusätzliche, weitergehende Informationen bereitzustellen.

In einem Schritt S1 werden von der Steuereinrichtung über den Kommunikationsweg Steuerinformationen an die Komponenten des rotierbaren Anteils, die ansteuerbar sind, gesendet, um die Rohdatenerfassung mit dem wenigstens einen Detektor vorzubereiten. Die Steuerinformationen umfassen dabei zum einen Aufbereitungsparameter, die angeben, wie die Rohdaten des wenigstens einen Röntgendetektors zu Detektordaten, die an die Recheneinrichtung zu übertragen sind, aufbereitet werden sollen. Andererseits enthalten die Steuerinformationen jedoch auch Selektionsparameter zur Parametrierung einer Selektionseinheit der Aufbereitungseinrichtung, in welcher die in jedem Zeitschritt entstehenden Detektordaten in einen ersten Datenstrom, der sofort an die Recheneinrichtung zu übertragen ist, und einen zweiten Datenstrom, dessen zweite Detektordaten in einer Zwischenspeichereinrichtung, die hier in die Aufbereitungseinrichtung integriert ist, gespeichert werden und erst nach Beendigung des ersten Datenstroms und der Rohdatenerfassung an die Recheneinrichtung übermittelt werden. Die Aufbereitungsparameter und die Selektionsparameter sind dabei aufeinander abgestimmt, insbesondere auch derart, dass die Datenübertragungsrate des ersten Datenstroms möglichst nah an die Maximalübertragungsrate der drahtlosen Kommunikationsstrecke herankommt. Eine die Aufteilung in die Datenströme beschreibende Selektionsinformation wird auch an die Recheneinrichtung übermittelt, so dass dort in einem Vorbereitungsschritt empfangenen Detektordaten letztlich kontinuierlich so sortiert (und gegebenenfalls dekomprimiert) werden können, dass sie von folgenden Rekonstruktionsschritten verarbeitet werden können, mithin in einem gewünschten Format geliefert werden. So benötigen die Rekonstruktionsschritte selbst keinerlei Wissen über die spezielle, hier beschriebene Datenübertragung.

Die Aufbereitungsparameter und die Selektionsparameter sowie auch die Selektionsinformation können hierbei einem gewählten Aufnahmeprotokoll für den Bildgebungsvorgang zugeordnet sein, beispielsweise mittels einer Look-up-Tabelle in der Steuereinrichtung. Die zugeordnete Steuerinformation umfasst dabei üblicherweise auch weitere Erfassungsparameter für die Rohdatenerfassung.

In einem Schritt S2 beginnt dann die Rohdatenerfassung, wonach für jeden Zeitschritt, in dem der wenigstens eine Röntgendetektor Rohdaten misst, diese im Schritt S3 durch die Aufbereitungseinrichtung aufbereitet werden, um Detektordaten zu ermitteln. Hierzu kann die Aufbereitungseinrichtung Aufbereitungseinheiten aufweisen, die beispielsweise neben einer Herstellung eines gewünschten Formats und einer gewünschten Sortierung auch eine Datenkompression durchführen können und/oder die räumliche Auflösung zumindest teilweise reduzieren können und dergleichen.

In einem Schritt S4 teilt die Selektionseinheit der Aufbereitungseinrichtung den eingehenden Detektordatenstrom in den ersten Datenstrom und den zweiten Datenstrom auf, wozu beispielsweise mittels der Selektionsparameter parametrierte Selektionskriterien verwendet werden können. Beispiele für derartige Selektionskriterien und wie unterhalb der Maximalübertragungsrate geblieben werden kann, werden im Folgenden noch genauer diskutiert.

Die Schritte S5 und S6 laufen dann parallel ab. Gemäß dem Schritt S5 wird der erste Datenstrom mit den ersten Detektordaten, insbesondere ein Datenpaket für jeden Zeitschritt, unmittelbar über den Kommunikationsweg an die Recheneinrichtung weiter übertragen. Eine Pufferung über eine kleinen Pufferspeicher, der ein erneutes Senden einzelner Datenpakete erlaubt, erfolgt allenfalls bei einem Übertragungsfehler. Somit ist eine Echtzeit-Übertragung gegeben. Der Schritt S5 kann durch eine Übertragungseinheit der Aufbereitungseinrichtung erfolgen. Parallel dazu wird im Schritt S6 mittels einer Zwischenspeichereinheit der zweite Datenstrom mit den zweiten Detektordaten in die Zwischenspeichereinrichtung weitergleitet und dort zwischengespeichert. Dabei kann im Übrigen auch der zum Schritt S5 erwähnte kleine Pufferspeicher als kleiner Anteil der Zwischenspeichereinrichtung vorgesehen sein.

In einem Schritt S7 wird dann überprüft, ob noch weitere Zeitschritte folgen oder ob die Rohdatenerfassung beendet ist. Folgen noch weitere Zeitschritte, wird bei Schritt S3 entsprechend fortgefahren, ist die Rohdatenerfassung beendet, werden im Schritt S8, nachdem auch die Übertragung des ersten Datenstroms abgeschlossen ist, die zweiten Detektordaten aus der Zwischenspeichereinrichtung mittels der Übertragungseinheit als Nachlauf zu der Recheneinrichtung übertragen. Hierbei wird idealerweise ebenso die Maximalübertragungsrate möglichst weitgehend ausgenutzt.

In Ausführungsbeispielen ist es denkbar, dass während des Schrittes S8 auch bereits schon Steuerinformationen zur Vorbereitung des nächsten Bildgebungsvorgangs an die Komponenten des rotierenden Anteils gesendet werden, falls dies nicht die Übertragung der zweiten Detektordaten verlangsamt und/oder stören könnte. Der kleine Pufferspeicher kann selbstverständlich auch für die zweiten Detektordaten verwendet werden.

Im Schritt S9 wird seitens der Recheneinrichtung der Vorverarbeitungsschritt, wie bereits erwähnt, durchgeführt, indem empfangene Detektordaten unter Verwendung der Selektierungsinformation sortiert werden und in ein Format gebracht werden, in dem nachfolgende Rekonstruktionsschritte die Detektordaten erwarten. Diese nachfolgenden Rekonstruktionsschritte nutzen im Schritt S10 die entsprechend vorbereiteten Detektordaten zur Rekonstruktion eines Computertomographiebilddatensatzes.

Dabei sei hervorgehoben, dass die Schritte S9 und S10 zumindest teilweise auch während der Übertragung der Detektordaten bereits durchgeführt werden können. Beispielsweise ist es denkbar, nach Erhalt der ersten Detektordaten zunächst einen vorläufigen Computertomographiebilddatensatz zu rekonstruieren und anzuzeigen, welcher dann mit Erhalt zweiter Detektordaten nach Abschluss der Übertragung oder sogar während der Übertragung schrittweise aktualisiert und aufgrund der weiteren Informationen verbessert wird.

Dabei sei in diesem Zusammenhang noch angemerkt, dass bei Ermittlung eines Computertomographiebilddatensatzes alleine aus den ersten Detektordaten, wenn für diese durch Weglassen in räumlicher, zeitlicher und/oder raumfrequenzbezogener Hinsicht die Maximalübertragungsrate eingehalten wird, eine Interpolation erfolgen kann, beispielsweise durch lineare Regression, aber auch durch komplexere Methoden, beispielsweise unter Nutzung künstlicher Intelligenz.

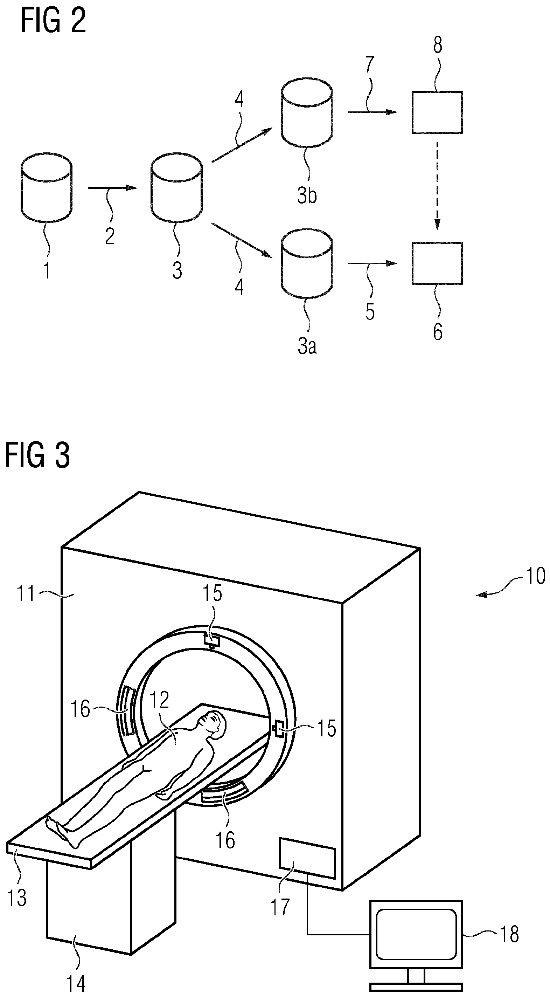

Fig. 2 zeigt erläuternd den Datenfluss gemäß dem Verfahren der Fig. 1. Dort entstehen an dem wenigstens einen Detektor zunächst Rohdaten 1, die durch Aufbereitung in der Aufbereitungseinrichtung, Pfeil 2, zu einer Gesamtheit von Detektordaten 3 aufbereitet werden. In der Selektionseinheit der Aufbereitungseinrichtung werden die Detektordaten 3 gemäß den Pfeilen 4 in erste Detektordaten 3a des ersten Datenstroms und zweite Detektordaten 3b des zweiten Datenstroms aufgeteilt. Die ersten Detektordaten 3a werden gemäß dem Pfeil 5 unmittelbar an die Recheneinrichtung 6 übertragen, während die zweiten Detektordaten 3b als zweiter Datenstrom gemäß dem Pfeil 7 in die Zwischenspeichereinrichtung 8 weitergeleitet werden, von wo sie nach Abschluss der Rohdatenerfassung gemäß dem gestrichelten Pfeil 9 dann ebenso an die Recheneinrichtung 6 übertragen werden können.

Die Aufteilung in den ersten Datenstrom und den zweiten Datenstrom (Pfeile 4, 5 beziehungsweise 4, 7) kann, je nach konkreter Bildgebungsaufgabe, aufgrund einer spektralen Sortierung, einer zeitlichen Sortierung, einer räumlichen Sortierung und/oder einer raumfrequenzbasierten Sortierung erfolgen. Wird als Detektor bevorzugt ein zählender Röntgendetektor, der auch die Energie von einzelnen Röntgenquanten quantifizieren kann, verwendet, kann die Rohdatenmessung beispielsweise für mehrere Energieschwellwerte erfolgen, wobei nur Detektordaten 3 eines Teils der Energieschwellwerte, insbesondere des wenigstens einen niedrigsten Energieschwellwerts, in den ersten Datenstrom selektiert werden können; die Detektordaten 3 anderer Energieschwellwerte werden in den zweiten Datenstrom einsortiert. Bei einer zeitlichen Sortierung kann beispielsweise nur das Ausleseergebnis des n-ten Zeitschritts übertragen werden. Bei der räumlichen Sortierung sind mehrere Ausgestaltungen denkbar. Zum einen können Detektordaten 3 mit niedrigerer räumlicher Auflösung ermittelt werden und als erste Detektordaten 3a sofort übertragen werden, während hochaufgelöste Detektordaten 3 als zweite Detektordaten 3b im Nachlauf übertragen werden. Möglich ist aber eine räumliche Sortierung auch durch vorläufiges Weglassen von Detektordaten 3, so dass beispielsweise in den ersten Datenstrom nur eine Auswahl verfügbarer Bildzeilen selektiert werden müssen. Dabei müssen für den ersten Datenstrom ausgewählte Untergruppen einer räumlichen Gesamtabdeckung nicht für jeden Zeitschritt gleich sein, sondern können sich auch beispielsweise von Zeitschritt zu Zeitschritt abwechseln beziehungsweise einer vorgegebenen Reihenfolge der Untergruppen folgen.

Hinsichtlich der Raumfrequenzen kann die Aufbereitungseinrichtung eine Transformationseinheit zur Ermittlung einer Raumfrequenzdarstellung der Detektordaten 3 aufweisen, so dass beispielsweise nur die Detektordaten 3 in den ersten Datenstrom selektiert werden können, deren Raumfrequenz unterhalb einer Raumfrequenzschwelle liegt. Es können auch Übertragungsverfahren, wie sie im Stand der Technik Video-Streaming eingesetzt werden, verwendet werden, beispielsweise eine Zerlegung entsprechend der vorkommenden Raumfrequenzen (Wavelets), so dass auf Empfängerseite durch Interpolation ein nutzbarer Detektordatensatz entsteht, der mit den nachlaufenden Datenteilen, also den zweiten Detektordaten 3b, zu einem vollständigen Datensatz zusammengefasst werden kann. Wie bereits erwähnt, kann eine Interpolation beispielsweise durch einfache lineare Regression erfolgen, denkbar sind jedoch auch komplexere Ansätze, beispielsweise solche unter Einsatz künstlicher Intelligenz. Ein Beispiel hierfür ist unter dem Schlagwort "Super-Resolution" bekannt.

In einem sehr konkreten Beispiel kann der Röntgendetektor beispielsweise eine Detektorfläche von 2752 x 288 Pixeln aufweisen, wobei zwei Energieschwellen in dem zählenden Röntgendetektor verwendet werden. Die entstehende Datenmenge ist deutlich zu groß für die unmittelbare Übertragung. Daher werden in der Aufbereitungseinrichtung Detektordaten 3 mit geringerer räumlicher Auflösung, beispielsweise 1376 x 144 Pixel, für die beiden Energieschwellwerte ermittelt. Diese ersten Detektordaten 3a werden in den ersten Datenstrom selektiert und sofort übertragen. Zusätzlich liegen die räumlich hochaufgelösten Detektordaten als zweite Detektordaten 3b in der Zwischenspeichereinrichtung 8 vor und können im Nachlauf übertragen werden. Dabei kann es ausreichend sein, die Detektordaten 3b für einen einzigen der Energieschwellwerte hoher räumlicher Auflösung zu übertragen. Dann liegen auf der Recheneinrichtung 6 die Detektordaten 3 derart vor, dass Detektordaten 3 des niedrigeren Energieschwellwerts einmal in hoher und einmal in niedriger räumlicher Auflösung vorhanden sind, Detektordaten des oberen Energieschwellwerts jedoch lediglich in der reduzierten räumlichen Auflösung.

Selbstverständlich sind auch Variationen denkbar, in denen beispielsweise zunächst nur Teildaten des einen Energieschwellwerts übertragen werden und dergleichen.

Fig. 3 zeigt eine Prinzipskizze einer erfindungsgemäßen Computertomographieeinrichtung 10, die zur Durchführung des erfindungsgemäßen Verfahrens ausgebildet ist.

Die Computertomographieeinrichtung 10 umfasst eine Gantry 11, die eine mittige Öffnung aufweist, in die ein Patient 12 mittels einer Patientenliege 13 eines Patiententisches 14 eingefahren werden kann. Der rotierende Anteil umfasst zwei hier nur angedeutete Aufnahmeanordnungen mit jeweils einem Röntgenstrahler 15 und jeweils einem zählenden Röntgendetektor 16. Es handelt sich mithin um eine Biplan-Computertomographieeinrichtung 10, die zur spektralen Bildgebung ausgebildet ist. Der Betrieb der Computertomographieeinrichtung 10 wird von einer Steuereinrichtung 17 gesteuert, der auch eine Anzeigeeinrichtung 18 zugeordnet sein kann und die die Recheneinrichtung 6 umfassen kann.

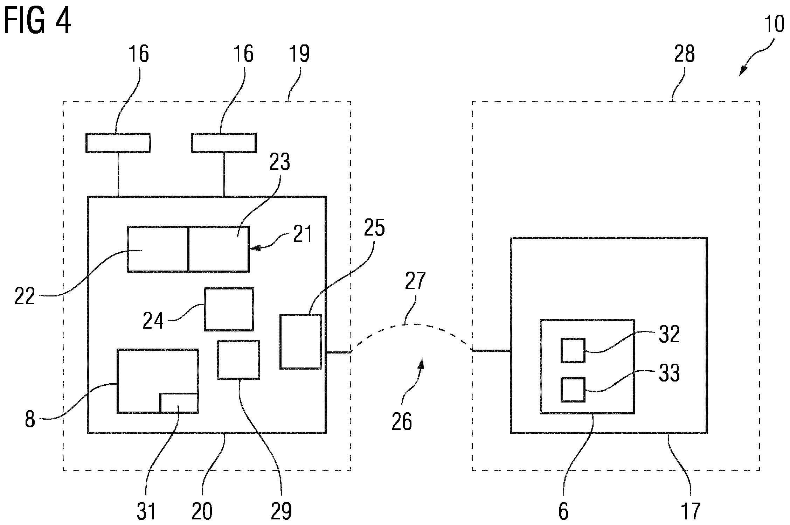

Hierzu ist die funktionale Struktur der Computertomographieeinrichtung 10 in Fig. 4 nochmals in vereinfachter Form dargestellt.

Wie erwähnt, umfasst die Computertomographieeinrichtung 10 einen rotierenden Anteil 19, zu dem auch die beiden Röntgendetektoren 16 gehören. Diese liefern ihre Rohdaten an die Aufbereitungseinrichtung 20, die neben Aufbereitungseinheiten 21 - beispielsweise umfassend die Transformationseinheit 22 und eine räumliche Auflösungsreduzierungseinheit 23 - die Selektionseinheit 24 umfasst. Während also die Aufbereitung der Rohdaten zu den Detektordaten 3 gemäß Schritt S3 durch die Aufbereitungseinheiten 21 erfolgt, erfolgt die Selektion und Aufteilung der Detektordaten 3 auf die Datenströme gemäß Schritt S4 in der Selektionseinheit 24. Der erste Datenstrom wird zu einer Übertragungseinheit 25 weitergeleitet, die die ersten Detektordaten 3a unmittelbar über den Kommunikationsweg 26 mit der drahtlosen Kommunikationsstrecke 27 zu der Steuereinrichtung 17 in den feststehenden Anteil 28 überträgt, und somit auch zur Recheneinrichtung 6, die einen Teil der Steuereinrichtung 17 bildet. Mit anderen Worten führt die Übertragungseinheit 25 den Schritt S5 aus.

Der zweite Datenstrom wird zu einer Zwischenspeichereinheit 29 weitergeleitet, welche die zweiten Detektordaten 3b in der Zwischenspeichereinrichtung 8 der Aufbereitungseinrichtung 20 gemäß Schritt S6 zwischenspeichert. Dabei sei an dieser Stelle angemerkt, dass ein Teil der Zwischenspeichereinrichtung 8 auch als Pufferspeicher 31 für ein erneutes Senden von Datenpaketen für die Übertragungseinheit 25 reserviert sein kann.

Mit Abschluss der Rohdatenerfassung und der Übertragung des ersten Datenstroms an die Recheneinrichtung 6 kann die Übertragungseinheit 25 auch die zweiten Detektordaten 3b über den Kommunikationsweg 26 an die Recheneinrichtung 6 übertragen (Schritt S8).

Die Recheneinrichtung 6 kann beispielsweise eine Vorbereitungseinheit 32 zur Durchführung des Vorbereitungsschrittes S9 sowie eine Rekonstruktionseinheit 33 zur Durchführung des Schrittes S10, mithin Rekonstruktion eines Computertomographiebilddatensatzes, aufweisen.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

Unabhängig vom grammatikalischen Geschlecht eines bestimmten Begriffes sind Personen mit männlicher, weiblicher oder anderer Geschlechteridentität mit umfasst.
werden.

## Patentansprüche

1. Verfahren zum Betrieb einer Computertomographieeinrichtung (10), welche einen rotierbaren Anteil (19) mit wenigstens einem Röntgendetektor (16) und einen gegenüber dem rotierbaren Anteil (19) feststehenden Anteil (28) mit einer Recheneinrichtung (6), die zur Verarbeitung von mit dem wenigstens einen Röntgendetektor (16) aufgenommenen Detektordaten (3, 3a, 3b) ausgebildet ist, aufweist, wobei dem wenigstens einen Röntgendetektor (16) eine Aufbereitungseinrichtung (20) zur Ermittlung von über einen Kommunikationsweg (26) zu der Recheneinrichtung (6) zu übertragenden Detektordaten (3, 3a, 3b) aus Rohdaten (1) des wenigstens einen Röntgendetektors (16) zugeordnet ist und der Kommunikationsweg (26) eine drahtlose Kommunikationsstrecke (27) mit einer Maximalübertragungsrate aufweist,
wobei bei einem Bildgebungsvorgang
- mittels einer Selektionseinheit (24) der Aufbereitungseinrichtung (20) die Detektordaten (3, 3a, 3b) in einen ersten Datenstrom, dessen erste Detektordaten (3a) nach Abschluss der Rohdatenerfassung mit dem wenigstens einen Röntgendetektor (16) zur Rekonstruktion eines auswertbaren Computertomographiebilddatensatzes ausreichend sind, mit einer Datenübertragungsrate, die höchstens der Maximalübertragungsrate entspricht, und einen zweiten Datenstrom, der als zweite Detektordaten (3b) die restlichen Detektordaten (3b) umfasst, aufgeteilt wird,
- der erste Datenstrom als Echtzeitübertragung unmittelbar über den Kommunikationsweg (26) an die Recheneinrichtung (6) übermittelt wird und
- die zweiten Detektordaten (3b) in einer Zwischenspeichereinrichtung (8) zwischengespeichert und, nach Abschluss der Rohdatenerfassung mit dem wenigstens einen Röntgendetektor (16) und der Übertragung des ersten Datenstroms, über den Kommunikationsweg (26) an die Recheneinrichtung (6) übertragen werden
- wobei die Selektionseinheit (24) die Aufteilung gemäß wenigstens einem, von einer Steuereinrichtung (17) der Computertomographieeinrichtung (10) vorgebbaren, Selektionsparameter vornimmt.

2. Verfahren nach Anspruch 1, **dadurch gekennzeichnet, dass** der erste Datenstrom wenigstens teilweise in der räumlichen Auflösung und/oder der räumlichen Abdeckung gegenüber den gesamten Detektordaten (3) reduzierte erste Detektordaten (3a) umfasst.

3. Verfahren nach Anspruch 2, **dadurch gekennzeichnet, dass** eine vorgegebene räumliche Gesamtabdeckung der gesamten Detektordaten (3) in wenigstens zwei Untergruppen zerlegt wird, wobei die Rohdatenerfassung in Zeitschritten erfolgt und in aufeinanderfolgenden Zeitschritten jeweils nur die Detektordaten (3, 3a, 3b) einer der Untergruppen gemäß einer vorgegebenen Reihenfolge der Untergruppen in den ersten Datenstrom selektiert werden.

4. Verfahren nach einem der vorangehenden Ansprüche, **dadurch gekennzeichnet, dass** die zeitliche Auflösung der ersten Detektordaten (3a) geringer als die der gesamten Detektordaten (3) ist.

5. Verfahren nach einem der vorangehenden Ansprüche, **dadurch gekennzeichnet, dass** die Aufbereitungseinrichtung (20) eine Transformationseinheit (22) zur Ermittlung einer Raumfrequenzdarstellung der Detektordaten (3, 3a, 3b) aufweist, wobei nur Detektordaten (3, 3a, 3b) mit Raumfrequenzen, die ein Raumfrequenzkriterium erfüllen, in den ersten Datenstrom einsortiert werden.

6. Verfahren nach einem der vorangehenden Ansprüche, **dadurch gekennzeichnet, dass**, falls Detektordaten (3, 3a, 3b) räumlich und/oder zeitlich und/oder frequenzbasiert für den ersten Datenstrom ausgelassen werden, bei Rekonstruktion eines Computertomographiebilddatensatzes aus den Detektordaten (3a) des ersten Datenstroms alleine wenigstens ein Teil der ausgelassenen Detektordaten (3b) durch Interpolation und/oder Extrapolation rekonstruiert wird.

7. Verfahren nach einem der vorangehenden Ansprüche, **dadurch gekennzeichnet, dass** die Computertomographieeinrichtung (10) zur spektralen Bildgebung ausgebildet ist und die Detektordaten (3, 3a, 3b) unterschiedlichen Energieparameterwerten zugeordnete Teildaten umfassen, wobei der erste Datenstrom nur für einen Teil der mehreren Energieparameterwerte Teildaten enthält.

8. Verfahren nach einem der vorangehenden Ansprüche, **dadurch gekennzeichnet, dass** die Aufteilung zur möglichst weitgehenden Nutzung der Maximalübertragungsrate vorgenommen wird.

9. Verfahren nach einem der vorangehenden Ansprüche, **dadurch gekennzeichnet, dass** jedem auswählbaren, Erfassungsparameter für die Rohdatenerfassung und/oder Aufbereitungsparameter für die Aufbereitung vorgebenden Aufnahmeprotokoll der wenigstens eine entsprechende Selektionsparameter zugeordnet ist und vor Start der Rohdatenerfassung an die Aufbereitungseinrichtung (20) übermittelt und dort eingestellt wird.

10. Verfahren nach einem der vorangehenden Ansprüche, **dadurch gekennzeichnet, dass** der wenigstens eine Selektionsparameter und/oder eine die Aufteilung in die Datenströme beschreibende Selektionsinformation auch an die Recheneinrichtung (6) übermittelt wird, wo in einem Vorbereitungsschritt die empfangenen Detektordaten (3, 3a, 3b) des ersten Datenstroms und des zweiten Datenstroms zu einem für folgende Datenverarbeitungsprozesse der Recheneinrichtung (6) als Eingangsdaten geeigneten Detektordatensatz kombiniert werden.

11. Verfahren nach einem der vorangehenden Ansprüche, **dadurch gekennzeichnet, dass** für Übertragungen über den Kommunikationsweg (26) ein Pufferspeicher (31) verwendet wird, der ein erneutes Senden eines Datenpakets bei einer fehlerhaften Übertragung erlaubt, wobei der Pufferspeicher (31) als ein hierfür reservierter Teil der Zwischenspeichereinrichtung (8) realisiert wird.

12. Verfahren nach einem der vorangehenden Ansprüche, **dadurch gekennzeichnet, dass** während der Übertragung der zweiten Detektordaten (3b) bereits eine den nächsten Bildgebungsvorgang vorbereitende Information an den rotierenden Anteil (19) übermittelt wird.

13. Verfahren nach einem der vorangehenden Ansprüche, **dadurch gekennzeichnet, dass** bei einer aufgrund eines Fehlers, insbesondere eines Systemausfalls, nur teilweisen Übertragung der zweiten Detektordaten (3b), die eine Zusatzinformation gegenüber den ersten Detektordaten (3a) umfassen, die Recheneinrichtung (6) zur Hinzufügung der Zusatzinformation in dem Computertomographiebilddatensatz wenigstens hinsichtlich des übertragenen Teils ausgebildet ist.

14. Computertomographieeinrichtung (10), welche einen rotierbaren Anteil (19) mit wenigstens einem Röntgendetektor (16) und einen gegenüber dem rotierbaren Anteil (19) feststehenden Anteil (28) mit einer Recheneinrichtung (6), die zur Verarbeitung von mit dem wenigstens einen Röntgendetektor (16) aufgenommenen Detektordaten (3, 3a, 3b) ausgebildet ist, und eine Steuereinrichtung (17) aufweist, wobei dem wenigstens einen Röntgendetektor (16) eine Aufbereitungseinrichtung (20) zur Ermittlung von über einen Kommunikationsweg (26) zu der Recheneinrichtung (6) zu übertragenden Detektordaten (3, 3a, 3b) aus Rohdaten (1) des wenigstens einen Röntgendetektors (16) zugeordnet ist und der Kommunikationsweg (26) eine drahtlose Kommunikationsstrecke (27) mit einer Maximalübertragungsrate aufweist,
wobei die Aufbereitungseinrichtung (20) folgende Einheiten aufweist :
- eine Selektionseinheit (24) zur Aufteilung der Detektordaten (3, 3a, 3b) eines Bildgebungsvorgangs in einen ersten Datenstrom, dessen erste Detektordaten (3a) nach Abschluss der Rohdatenerfassung mit dem wenigstens einen Röntgendetektor (16) zur Rekonstruktion eines auswertbaren Computertomographiebilddatensatzes ausreichend sind, mit einer Datenübertragungsrate, die höchstens der Maximalübertragungsrate entspricht, und einen zweiten Datenstrom, der als zweite Detektordaten (3b) die restlichen Detektordaten (3b) umfasst, gemäß wenigstens einem, von der Steuereinrichtung (17) vorgebbaren, Selektionsparameter,
- eine Zwischenspeichereinheit (29) zum Zwischenspeichern der zweiten Detektordaten (3b) des zweiten Datenstroms in einer Zwischenspeichereinrichtung (8), und
- eine Übertragungseinheit (25) zur unmittelbaren Echtzeitübertragung des ersten Datenstroms über den Kommunikationsweg (26) an die Recheneinrichtung (6) und, nach Abschluss der Rohdatenerfassung des Bildgebungsvorgangs, zur Übertragung der zweiten Detektordaten (3b) über den Kommunikationsweg (26) an die Recheneinrichtung (6).

## Claims

1. Method for operating a computed tomography facility (10) which has a rotatable portion (19) with at least one X-ray detector (16) and a portion (28) which is fixed relative to the rotatable portion (19) with a computing facility (6) embodied to process detector data (3, 3a, 3b) recorded with the at least one X-ray detector (16), wherein the at least one X-ray detector (16) is assigned a preparation facility (20) for ascertaining detector data (3, 3a, 3b) to be transmitted to the computing facility (6) via a communication path (26) from raw data (1) of the at least one X-ray detector (16) and the communication path (26) has a wireless communication link (27) with a maximum transmission rate,
wherein, during an imaging process,
- a selection unit (24) of the preparation facility (20) divides the detector data (3, 3a, 3b) into a first data stream with first detector data (3a) that is sufficient for the reconstruction of an evaluable computed tomography image dataset after completion of the raw data acquisition with the at least one X-ray detector (16) with a data transmission rate corresponding at most to the maximum transmission rate and a second data stream comprising the remaining detector data (3b) as second detector data (3b),
- the first data stream is transferred as a real-time transmission directly to the computing facility (6) via the communication path (26) and
- the second detector data (3b) is temporarily stored in a temporary storage facility (8) and, after completion of the raw data acquisition with the at least one X-ray detector (16) and the transmission of the first data stream, is transmitted to the computing facility (6) via the communication path (26)
- wherein the selection unit (24) carries out the division according to at least one selection parameter which can be prespecified by a control facility (17) of the computed tomography facility (10).

2. Method according to claim 1, **characterised in that** the first data stream comprises first detector data (3a) that is at least partially reduced in spatial resolution and/or spatial coverage compared to the entire detector data (3).

3. Method according to claim 2, **characterised in that** a prespecified overall spatial coverage of the entire detector data (3) is split into at least two subgroups, wherein the raw data acquisition takes place in time steps and in each case only the detector data (3, 3a, 3b) of one of the subgroups is selected into the first data stream in successive time steps according to a prespecified sequence of the subgroups.

4. Method according to one of the preceding claims, **characterised in that** the temporal resolution of the first detector data (3a) is lower than that of the entire detector data (3).

5. Method according to one of the preceding claims, **characterised in that** the preparation facility (20) has a transformation unit (22) for ascertaining a spatial frequency representation of the detector data (3, 3a, 3b), wherein only detector data (3, 3a, 3b) with spatial frequencies that satisfy a spatial frequency criterion is sorted into the first data stream.

6. Method according to one of the preceding claims, **characterised in that**, if detector data (3, 3a, 3b) is omitted spatially and/or temporally and/or frequency-based for the first data stream, on the reconstruction of a computed tomography image dataset from the detector data (3a) of the first data stream alone, at least a part of the omitted detector data (3b) is reconstructed by interpolation and/or extrapolation.

7. Method according to one of the preceding claims, **characterised in that** the computed tomography facility (10) is embodied for spectral imaging and the detector data (3, 3a, 3b) comprises partial data assigned to different energy parameter values, wherein the first data stream only contains partial data for some of the plurality of energy parameter values.

8. Method according to one of the preceding claims, **characterised in that** the division is carried out in order to make the greatest possible use of the maximum transmission rate.

9. Method according to one of the preceding claims, **characterised in that** the at least one corresponding selection parameter is assigned to each selectable recording protocol specifying acquisition parameters for the raw data acquisition and/or preparation parameters for the preparation and transferred to the preparation facility (20) and set there before the start of the raw data acquisition.

10. Method according to one of the preceding claims, **characterised in that** the at least one selection parameter and/or selection information describing the division into the data streams is also transferred to the computing facility (6) when, in an initialisation step, the received detector data (3, 3a, 3b) of the first data stream and the second data stream is combined to form a detector dataset suitable as input data for subsequent data processing operations of the computing facility (6).

11. Method according to one of the preceding claims, **characterised in that**, for transmissions via the communication path (26), a buffer memory (31) is used which allows a data packet to be resent in the event of a faulty transmission, wherein the buffer memory (31) is implemented as part of the temporary storage facility (8) reserved for this purpose.

12. Method according to one of the preceding claims, **characterised in that**, during the transmission of the second detector data (3b), initialisation information for the next imaging process is already transferred to the rotating portion (19).

13. Method according to one of the preceding claims, **characterised in that**, in the event of only partial transmission of the second detector data (3b), which comprises additional information compared to the first detector data (3a), due to an error, in particular a system failure, the computing facility (6) is embodied to add the additional information in the computed tomography image dataset at least with respect to the transmitted part.

14. Computed tomography facility (10) which has a rotatable portion (19) with at least one X-ray detector (16) and a portion (28) which is fixed relative to the rotatable portion (19) with a computing facility (6) embodied to process detector data (3, 3a, 3b) recorded with the at least one X-ray detector (16), and a control facility (17), wherein the at least one X-ray detector (16) is assigned a preparation facility (20) for ascertaining detector data (3, 3a, 3b) to be transmitted to the computing facility (6) via a communication path (26) from raw data (1) of the at least one X-ray detector (16) and the communication path (26) has a wireless communication link (27) with a maximum transmission rate, wherein the preparation facility (20) has the following units:
- a selection unit (24) for dividing the detector data (3, 3a, 3b) of an imaging process into a first data stream with first detector data (3a) that is sufficient for the reconstruction of an evaluable computed tomography image dataset after completion of the raw data acquisition with the at least one X-ray detector (16) with a data transmission rate corresponding at most to the maximum transmission rate and a second data stream comprising the remaining detector data (3b) as second detector data (3b), according to at least one selection parameter that can be prespecified by the control facility (17),
- a temporary storage unit (29) for temporarily storing the second detector data (3b) of the second data stream in a temporary storage facility (8), and
- a transmission unit (25) for direct real-time transmission of the first data stream via the communication path (26) to the computing facility (6) and, after completion of the raw data acquisition of the imaging process, for transmission of the second detector data (3b) via the communication path (26) to the computing facility (6).

## Revendications

1. Procédé pour faire fonctionner un dispositif (10) de tomodensitométrie assistée par ordinateur, qui a une partie (19) tournante ayant au moins un détecteur (16) de rayons X et ayant, fixe par rapport à la partie (19) tournante, une partie (28) ayant un dispositif (6) informatique, qui est constitué pour le traitement de données (3, 3a, 3b) de détecteur enregistrées par le au moins un détecteur (16) de rayons X, dans lequel le au moins un détecteur (16) de rayons X est associé à un dispositif (20) de préparation pour la détermination, à partir de données (1) brutes du au moins un détecteur (16) de rayons X, de données (3, 3a, 3b) de détecteur à transmettre au dispositif (6) informatique en passant par un chemin (26) de communication et le chemin (26) de communication a une section (27) de communication sans fil ayant un débit de transmission maximum,
dans lequel lors d'une opération d'imagerie
- au moyen d'une unité (24) de sélection du dispositif (20) de préparation, on répartit les données (3, 3a, 3b) de détecteur en un premier flux de données, dont les premières données (3a) de détecteur sont, après la fin de la détection de données brutes par le au moins un détecteur (16) de rayons X, suffisantes pour la reconstruction d'un ensemble exploitable de données d'image par tomodensitométrie assistée par ordinateur, à un débit de transmission de données, qui correspond au plus au débit de transmission maximum, et en un deuxième flux de données, qui comprend comme deuxièmes données (3b) de détecteur les données (3b) restantes de détecteur,
- on transmet le premier flux de données comme transmission en temps réel directement par le chemin (26) de communication au dispositif (6) informatique, et
- on met en mémoire tampon les deuxièmes données (3b) de détecteur dans un dispositif (8) de mémoire tampon et, après la fin de la détection de données brutes par le au moins un détecteur (16) de rayons X et la transmission du premier flux de données, on les transmet au dispositif (6) informatique en passant par le chemin (26) de communication,
- dans lequel l'unité (24) de sélection effectue la répartition suivant au moins un paramètre de sélection pouvant être donné à l'avance par un dispositif (17) de commande du dispositif (10) de tomodensitométrie assistée par ordinateur.

2. Procédé suivant la revendication 1, **caractérisé en ce que** le premier flux de données comprend des premières données (3) de détecteur réduites au moins en partie dans la résolution spatiale et/ou la couverture spatiale par rapport à l'ensemble (3a) des données de détecteur.

3. Procédé suivant la revendication 2, **caractérisé en ce que** l'on décompose un recouvrement spatial d'ensemble donné à l'avance de l'ensemble des données (3) de détecteur en au moins deux sous-groupes, dans lequel la détection de données brutes s'effectue dans des laps de temps et, dans des laps de temps successifs, on sélectionne, suivant une séquence donnée à l'avance des sous-groupes dans le premier flux de données, respectivement seulement les données (3, 3a, 3b) de détecteur de l'un des sous-groupes.

4. Procédé suivant l'une des revendications précédentes, **caractérisé en ce que** la résolution temporelle des premières données (3a) de détecteur est plus petite que celle de l'ensemble des données (3) de détecteur.

5. Procédé suivant l'une des revendications précédentes, **caractérisé en ce que** le dispositif (20) de préparation a une unité (22) de transformation, pour la détermination d'une représentation de fréquence spatiale des données (3, 3a, 3b) de détecteur, dans lequel on n'entre, dans le premier flux de données, que des données (3, 3a, 3b) de détecteur ayant des fréquences spatiales, qui satisfont un critère de fréquence spatiale.

6. Procédé suivant l'une des revendications précédentes, **caractérisé en ce que**, si l'on supprime des données (3, 3a, 3b) de détecteur sur une base spatiale et/ou temporelle et/ou fréquentielle pour le premier flux de données, lors de la reconstruction d'un ensemble de données d'image de tomodensitométrie assistée par ordinateur, à partir des données (3a) de détecteur du premier flux de données, on reconstruit par interpolation et/ou extrapolation seulement au moins une partie des données (3b) de détecteur supprimées.

7. Procédé suivant l'une des revendications précédentes, **caractérisé en ce que** le dispositif (10) de tomodensitométrie assistée par ordinateur est constitué pour l'imagerie spectrale et les données (3, 3a, 3b) de détecteur comprennent des données partielles associées à des valeurs de paramètres d'énergie différentes, dans lequel le premier flux de données ne contient que des données partielles pour une partie des plusieurs valeurs de paramètres d'énergie.

8. Procédé suivant l'une des revendications précédentes, **caractérisé en ce que** l'on effectue la répartition, pour l'utilisation aussi grande que possible du débit de transmission maximum.

9. Procédé suivant l'une des revendications précédentes, **caractérisé en ce que**, à chaque paramètre de détection pouvant être sélectionné pour la détection des données brutes et/ou à chaque paramètre de préparation pour la préparation pour le protocole d'enregistrement précédent, le au moins un paramètre de sélection correspondant est associé et est, avant le début de la détection de données brutes, transmis au dispositif (20) de préparation et y est établi.

10. Procédé suivant l'une des revendications précédentes, **caractérisé en ce que** l'on transmet le au moins un paramètre de sélection et/ou une information de sélection décrivant la répartition dans les flux de données également au dispositif (6) informatique, où dans un stade de traitement préalable, on combine les données (3, 3a, 3b) de détecteur reçues du premier flux de données et du deuxième flux de données en un ensemble de données de détecteur approprié comme données d'entrée pour des processus suivants de traitement de données du dispositif (6) informatique.

11. Procédé suivant l'une des revendications précédentes, **caractérisé en ce que** l'on utilise, pour des transmissions en passant par le chemin (26) de communication, une mémoire (31) tampon, qui permet un envoi renouvelé d'un paquet de données, lors d'une transmission défectueuse, dans lequel on réalise la mémoire (31) tampon sous la forme d'une partie réservée à cet effet du dispositif (8) de mémoire tampon.

12. Procédé suivant l'une des revendications précédentes, **caractérisé en ce que**, pendant la transmission des deuxièmes données (3b) de détecteur, on transmet déjà, à la partie (19) tournante, une information préparant l'opération suivante d'imagerie.

13. Procédé suivant l'une des revendications précédentes, **caractérisé en ce que**, lors d'une transmission seulement en partie en raison d'une erreur, en particulier d'une défaillance du système, des deuxièmes données (3b) de détecteur, qui comprennent une information supplémentaire par rapport aux premières données (3a) de détecteur, le dispositif (6) informatique est constitué pour ajouter l'information supplémentaire à l'ensemble de données d'image de tomodensitométrie assistée par ordinateur, au moins en ce qui concerne la partie transmise.

14. Dispositif (10) de tomodensitométrie assistée par ordinateur, qui a une partie (19) tournante ayant au moins un détecteur (16) de rayons X et ayant, fixe par rapport à la partie (19) tournante, une partie (28) ayant un dispositif (6) informatique, qui est constitué pour le traitement de données (3, 3a, 3b) de détecteur enregistrées par le au moins un détecteur (16) de rayons X, et un dispositif (17) de commande, dans lequel le au moins un détecteur (16) de rayons X est associé à un dispositif (20) de préparation pour la détermination, à partir de données (1) brutes du au moins un détecteur (16) de rayons X, de données (3, 3a, 3b) de détecteur à transmettre au dispositif (6) informatique en passant par un chemin (26) de communication et le chemin (26) de communication a une section (27) de communication sans fil ayant un débit de transmission maximum,
dans lequel le dispositif (20) de préparation a les unités suivantes :
- une unité (24) de sélection pour la répartition des données (3, 3a, 3b) de détecteur d'une opération d'imagerie en un premier flux de données, dont les premières données (3a) de détecteur sont, après la fin de la détection de données brutes par le au moins un détecteur (16) de rayons X, suffisantes pour la reconstruction d'un ensemble exploitable de données d'image par tomodensitométrie assistée par ordinateur, à un débit de transmission de données, qui correspond au plus au débit de transmission maximum, et en un deuxième flux de données, qui comprend comme deuxièmes données (3b) de détecteur les données (3b) restantes de détecteur, suivant au moins un paramètre de sélection pouvant être donné à l'avance par le dispositif (17) de commande,
- une unité (29) de mémoire tampon pour la mise en mémoire tampon des deuxièmes données (3b) de détecteur du deuxième flux de données dans un dispositif (8) de mémoire tampon, et
- une unité (25) de transmission pour la transmission directe en temps réel du premier flux de données au dispositif (6) informatique en passant par le chemin (26) de communication et, après la fin de la détection de données brutes de l'opération d'imagerie, pour la transmission des deuxièmes données (3b) de détecteur au dispositif (6) informatique en passant par le chemin (26) de communication.
